# FASCICULE DE BREVET EUROPEEN

(11) **EP 4 572 825 B1**
(45) Date de publication et mention de la délivrance du brevet: **08.04.2026**
(21) Numéro de dépôt: 23768325.5
(22) Date de dépôt: 16.08.2023
(51) Int. Cl.: A61M 15/00

(54) **ENSEMBLE COMPORTANT UN INHALATEUR DE POUDRE SÈCHE ET UNE BOITE DE STOCKAGE**
SET MIT EINEM TROCKENPULVERINHALATOR UND EINER AUFBEWAHRUNGSBOX
SET COMPRISING A DRY POWDER INHALER AND A STORAGE BOX

(30) Priorité: 18.08.2022 FR 2208358
(43) Date de publication de la demande: 25.06.2025
(73) Titulaire: APTAR France SAS, 27110 Le Neubourg (FR)
(72) Inventeur: POULLAIN, Franck, 27400 LA HAYE MALHERBE (FR)
(74) Mandataire: CAPRI
(86) Numéro de dépôt international: PCT/FR2023/051268
(87) Numéro de publication internationale: WO 2024/038233

(56) Documents cités:
- WO-A1-03/030974
- WO-A1-2008/001132
- WO-A1-2013/008037
- CN-A- 107 469 206
- TW-A- 200 924 806

## Description

La présente invention concerne un ensemble comportant un inhalateur de poudre sèche et une boite de stockage.

Les inhalateurs de poudre sont bien connus dans l'état de la technique. Il en existe différentes sortes.

Un premier type d'inhalateur multidoses contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Ces dispositifs nécessitent des moyens de dosage complexes et donc coûteux, et la précision et la reproductibilité du dosage ne sont pas garanties. De plus, il y a des risques de contamination de la poudre disposée dans le réservoir au fur et à mesure de l'utilisation de l'inhalateur.

Un autre type d'inhalateur multidoses consiste à prévoir plusieurs réservoirs individuels contenant chacun une dose de poudre, puis d'ouvrir un de ces réservoirs à chaque actionnement de l'inhalateur. Cette mise en œuvre assure une meilleure étanchéité de la poudre, puisque chaque dose de poudre n'est exposée à l'atmosphère qu'au moment de son expulsion. Pour réaliser ces ensembles de réservoirs individuels, diverses variantes ont déjà été proposées, telle que des bandes ou disques de blisters. Concernant l'ouverture des réservoirs individuels, il a été proposé de peler ou décoller la couche de fermeture des blisters. Ceci présente l'inconvénient d'une maîtrise difficile des forces à appliquer pour garantir une ouverture totale sans risquer d'ouvrir le réservoir suivant, particulièrement si les moyens d'ouverture doivent être actionnés par l'inhalation. Une autre solution est de percer la membrane de fermeture d'un blister à chaque actionnement, ce qui requiert des moyens de perçage complexes et donc coûteux.

Les inhalateurs multidoses décrits ci-dessus présentent de plus le problème de ne pas garantir une efficacité de la distribution optimale, avec une partie importante de la dose qui pénètre effectivement dans les poumons de l'utilisateur pour avoir un effet thérapeutique bénéfique.

Pour réaliser des dispositifs moins complexes et donc moins coûteux, il a été proposé des inhalateurs unidoses comportant un seul réservoir individuel, tels qu'un blister ou une capsule, qui est à charger dans l'inhalateur juste avant l'utilisation de celui-ci. L'avantage de ces dispositifs unidoses est qu'il n'est pas nécessaire de stocker l'ensemble des doses à l'intérieur de l'appareil, de sorte que celui-ci peut être de dimension réduite. Par contre, l'utilisation est plus compliquée pour l'utilisateur, puisque celui-ci est obligé de charger un blister ou une capsule dans l'inhalateur avant chaque utilisation. De plus, d'autres inconvénients spécifiques à ces inhalateurs unidoses existent. Ainsi, un blister ou une capsule ne permet pas la distribution de grandes doses, typiquement supérieures à 100 mg. De plus, l'ouverture, notamment des capsules ou blisters à percer, impose l'utilisation de moyens de perçage complexes, ainsi qu'un risque de résidus de membrane percée dans la poudre distribuée. Par ailleurs, les performances de vidage du réservoir ne sont pas toujours optimales, une partie de la poudre pouvant rester à l'intérieur du réservoir lors de l'inhalation.

Pour tenter de pallier à ces inconvénients, il a été proposé dans le document WO9826828 un inhalateur unidose comportant un réservoir cylindrique pourvu d'une ouverture latérale, disposé dans une chambre cylindrique de plus grand diamètre, de sorte que lors de l'inhalation, le réservoir se déplace dans la chambre selon un mouvement orbital, ce qui provoque l'expulsion de la poudre. Cette mise en œuvre permet effectivement d'améliorer l'efficacité de la distribution, avec un vidage sensiblement total du réservoir et une partie plus importante de la dose qui pénètre effectivement dans les poumons. Néanmoins, un inconvénient de ce dispositif est qu'en cas de chute de l'inhalateur, le réservoir peut se déplacer de manière non souhaitée dans la chambre sous l'effet du choc, avec par conséquent un risque de perte de poudre ou de contamination.

Les documents WO2013008037A1, WO2008001132A1, TW200924806A, WO03030974A1 et CN107469206A décrivent d'autres dispositifs de l'état de la technique.

Le document WO 2013/008037 A1, par exemple, décrit un inhalateur de poudre sèche comportant un corps contenant une chambre et un embout buccal pourvu d'un orifice de distribution à travers lequel l'utilisateur inhale, un réservoir contenant une dose de poudre sèche à inhaler et comportant un orifice latéral de sortie, ledit réservoir étant déplaçable entre une position non chargée, dans laquelle il est au moins partiellement disposé hors de ladite chambre, et une position chargée, dans laquelle il est entièrement disposé dans ladite chambre, le diamètre dudit réservoir étant inférieur au diamètre de ladite chambre, un organe d'actionnement pour déplacer ledit réservoir de sa position non chargée vers sa position chargée. Le document WO 2013/008037 A1 divulgue également que cet inhalateur de poudre sèche est un dispositif jetable à usage unique qui est fourni dans un emballage en aluminium scellé afin d'empêcher la pénétration d'humidité.

Les documents WO 03/030974 A1 et CN 107 469 206 A décrivent différents types de boîtiers de stockage pour inhalateurs de poudre sèche.

La présente invention a pour but de fournir un ensemble qui ne reproduit pas les inconvénients susmentionnés.

En particulier, la présente invention a pour but de fournir un tel ensemble qui empêche tout risque de perte de poudre ou de contamination en cas de chute de l'inhalateur.

La présente invention a également pour but de fournir un tel ensemble qui limite voire empêche les risques de contamination et/ou de pollution de la poudre.

La présente invention a également pour but de fournir un tel ensemble qui réduit la complexité d'utilisation pour l'utilisateur et qui garantit une meilleure sécurité d'utilisation.

La présente invention a aussi pour but de fournir un tel ensemble qui soit simple et peu coûteux à fabriquer et à assembler.

La présente invention a donc pour objet un ensemble comportant :
- un inhalateur de poudre sèche comportant un corps contenant une chambre et un embout buccal pourvu d'un orifice de distribution à travers lequel l'utilisateur inhale, un réservoir contenant une dose de poudre sèche à inhaler et comportant un orifice latéral de sortie, ledit réservoir étant déplaçable entre une position non chargée, dans laquelle il est au moins partiellement disposé hors de ladite chambre, et une position chargée, dans laquelle il est entièrement disposé dans ladite chambre, le diamètre dudit réservoir étant inférieur au diamètre de ladite chambre, un organe d'actionnement pour déplacer ledit réservoir de sa position non chargée vers sa position chargée, et
- une boite de stockage comportant un corps creux borgne comportant une paroi de fond supportant au moins une projection axiale s'étendant axialement vers le haut, ladite au moins une projection axiale, en position de stockage avec ledit inhalateur disposé dans ladite boite de stockage, traversant ledit embout buccal et ladite chambre pour venir en contact dudit réservoir pour bloquer ledit réservoir dans sa position non chargée.

Avantageusement, ledit corps creux borgne est ouvert du côté supérieur et fermé par un couvercle.

Avantageusement, ledit réservoir est formé d'une partie supérieure de réservoir et d'une partie inférieure de réservoir, qui sont assemblées après remplissage avec la dose de poudre.

Avantageusement, ladite partie inférieure de réservoir comporte ledit orifice latéral de sortie.

Avantageusement, ladite au moins une projection axiale de ladite boite de stockage coopère en position de stockage avec ladite partie supérieure de réservoir.

Avantageusement, en position non chargée dudit réservoir, ledit orifice latéral de sortie dudit réservoir est obturé par un élément de fermeture solidaire dudit corps de l'inhalateur.

Selon une variante avantageuse, ledit réservoir comporte un élément de fermeture déplaçable et/ou déformable par rapport audit réservoir entre une position de fermeture dudit orifice latéral de sortie et une position d'ouverture dudit orifice latéral de sortie, ledit élément de fermeture étant en position de fermeture quand ledit réservoir est en position non chargée, et étant en position d'ouverture quand ledit réservoir est en position chargée.

Avantageusement, ladite chambre est disposée entre une entrée d'air d'inhalation et ledit embout buccal.

Avantageusement, des canaux tangentiels amènent un flux d'air d'inhalation de manière tangentielle dans ladite chambre, ce qui génère un déplacement orbital dudit réservoir dans ladite chambre.

Avantageusement, ledit réservoir contient une dose de poudre supérieure à 50 mg, notamment supérieure à 100 mg.

Avantageusement, le volume occupé par ledit réservoir dans ladite chambre en position chargée est supérieur à 50% du volume de ladite chambre.

Avantageusement, le diamètre radial dudit réservoir est supérieur à sa hauteur axiale.

Avantageusement, ledit organe d'actionnement est déplaçable axialement par rapport audit corps pour déplacer ledit réservoir de sa position non chargée vers sa position chargée.

Avantageusement, ledit organe d'actionnement comporte au moins une rainure oblique recevant au moins un ergot respectif formé sur ledit corps ou sur un élément solidaire dudit corps, de sorte que lors de son déplacement axial, ledit organe d'actionnement réalise une rotation dans ledit corps.

Ces caractéristiques et avantages et d'autres de la présente invention apparaîtront plus clairement au cours de la description détaillée suivante, faite en référence aux dessins joints, donnés à titre d'exemples non limitatifs, sur lesquels
la figure 1 est une vue schématique en section transversale d'un ensemble selon un premier mode de réalisation avantageux, en position verrouillée,
la figure 2 est une vue schématique en section transversale d'un inhalateur selon un second mode de réalisation avantageux, en position non chargée,
la figure 3 est une vue similaire à celle de la figure 2, en position chargée,
la figure 4 est une vue schématique de dessous du réservoir dans la chambre, en position chargée, et
la figure 5 est une vue schématique de dessus de l'embout buccal.

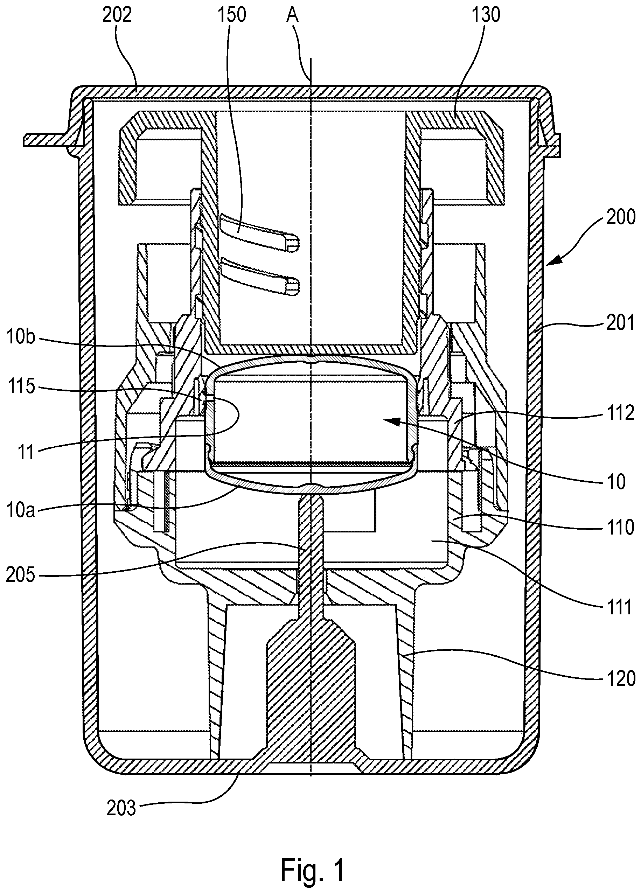

Dans la description ci-après, les termes "supérieur", "inférieur" et "latéral" se réfèrent à la position droite du dispositif représenté sur les figures 1 à 3. Les termes "axial" et "radial" se réfèrent à l'axe central longitudinal A du dispositif, représenté sur la figure 1.

Les figures décrivent deux modes de réalisation d'un inhalateur adapté à faire partie d'un ensemble selon la présente invention.

La figure 1 montre un premier mode de réalisation de l'inhalateur.

Dans ce premier mode de réalisation, l'inhalateur comporte un corps 110 contenant une chambre 111 et un embout buccal 120 pourvu d'un orifice de distribution 121 à travers lequel l'utilisateur inhale.

Avantageusement, le corps 110 est formé de deux parties de corps, une partie de corps supérieure comportant l'embout buccal 120 et une partie de corps inférieure comportant un manchon cylindrique 112, la chambre 111 étant définie par ces deux parties de corps fixées l'une à l'autre.

De préférence, la chambre 111 est disposée entre une entrée d'air d'inhalation et ledit embout buccal 120, de sorte que lorsque l'utilisateur inhale à travers l'embout buccal 120, le flux d'air va traverser ladite chambre 111.

L'inhalateur comporte un réservoir 10 contenant une dose de poudre sèche à inhaler et comportant un orifice latéral de sortie 11. Ce réservoir 10 est déplaçable entre une position non chargée, dans laquelle il est au moins partiellement disposé hors de ladite chambre 111, et une position chargée, dans laquelle il est entièrement disposé dans ladite chambre 111. Le réservoir 10 comporte un orifice latéral de sortie 11 obturé par un élément de fermeture 115 lorsque le réservoir 10 n'est pas dans sa position chargée. Avantageusement, c'est la partie inférieure de réservoir 10b qui comporte l'orifice latéral de sortie 11.

Dans ce premier mode de réalisation, l'élément de fermeture 115 est formé sur le manchon cylindrique 112.

Le diamètre du réservoir 10 est inférieur au diamètre de ladite chambre 111, de sorte qu'en position chargée, le réservoir 10 peut se déplacer dans la chambre 111. Avantageusement, des canaux tangentiels 115a, 115b, 115c amène ledit flux d'air d'inhalation de manière tangentielle dans la chambre 111, ce qui va provoquer un déplacement orbital du réservoir 10 dans la chambre 111, le réservoir 10 tournant sur lui-même tout en tournant autour de la périphérie de la chambre 111 le long de la paroi latérale de celle-ci. Ce mouvement va permettre l'expulsion de la poudre contenue dans le réservoir 10, qui est entrainée par le flux d'air d'inhalation vers l'embout buccal et l'orifice de distribution.

Avantageusement, le réservoir 10 peut contenir une dose de poudre supérieure à 50 mg, notamment supérieure à 100 mg. Eventuellement, des doses très importantes peuvent être envisagées, notamment supérieures à 500 mg.

Avantageusement, le volume occupé par le réservoir 10 dans la chambre 111 en position chargée est supérieur à 50% du volume de la chambre 111.

Avantageusement, le diamètre radial du réservoir 10 est supérieur à sa hauteur axiale.

Avantageusement, le réservoir 10 est formé d'une partie supérieure de réservoir 10a et d'une partie inférieure de réservoir 10b, qui sont assemblées après remplissage avec la dose de poudre.

L'inhalateur comporte aussi un organe d'actionnement 130 pour déplacer ledit réservoir 10 de sa position non chargée vers sa position chargée. Cet organe d'actionnement 130 est déplaçable axialement par rapport au corps 110 pour pousser le réservoir 10 de sa position non chargée vers sa position chargée.

Avantageusement, le déplacement axial de l'organe d'actionnement 130 se fait par une rotation de l'organe d'actionnement 130 dans le corps 110, par exemple au moyen d'ergot(s) du corps 110 disposé(s) dans une(des) rainure(s) oblique(s) de l'organe d'actionnement 130.

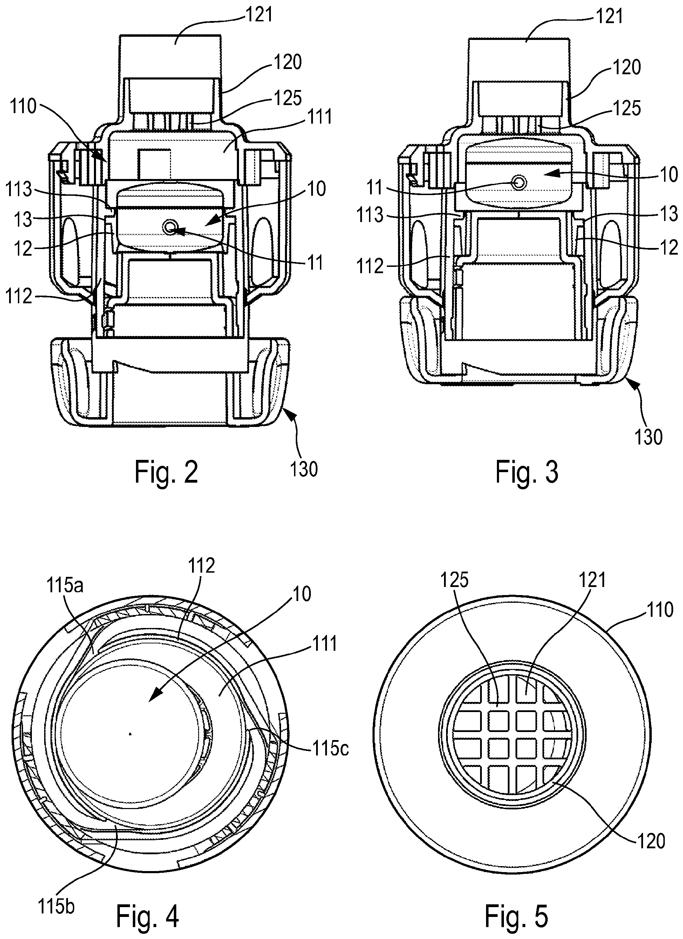

Les figures 2 et 3 montrent un second mode de réalisation de l'inhalateur.

Dans ce second mode de réalisation, l'orifice latéral de sortie 11 du réservoir est obturé par un élément de fermeture 12 spécifique qui s'adapte sur le réservoir, et qui ne fait pas partie de l'inhalateur, contrairement à l'élément de fermeture 115 de la figure 1.

Cet élément de fermeture 12 protège la poudre contenue dans le réservoir jusqu'à sa distribution par l'actionnement de l'inhalateur.

Ainsi, l'élément de fermeture 12 est déplaçable et/ou déformable par rapport au réservoir 10 entre des positions de fermeture et d'ouverture de l'orifice latéral de sortie 11, ledit élément de fermeture 12 étant en position de fermeture quand ledit réservoir 10 est en position non chargée, et étant en position d'ouverture quand ledit réservoir 10 est en position chargée.

Avantageusement, en position non chargée, l'élément de fermeture 12 est en butée sur une partie du corps 110 ou solidaire de celui-ci, de sorte que lorsque le réservoir 10 est déplacé vers sa position chargée, l'élément de fermeture 12 reste bloqué dans le corps, ouvrant ainsi l'orifice latéral de sortie 11.

Dans l'exemple des figures 2 et 3, l'élément de fermeture 12 comporte une projection radiale 13 qui coopère avec un épaulement 113 du manchon cylindrique 112.

Avantageusement, la projection radiale 13 est disposée d'un coté de l'élément de fermeture 12, de sorte que celui-ci n'est pas symétrique. Ainsi, lorsque l'élément de fermeture 12 est en position de fermeture sur le réservoir 10, celui-ci n'est pas non plus symétrique, ce qui forme un détrompage pour l'insertion du réservoir 10 dans le corps 110 de l'inhalateur, cette insertion n'étant possible que dans une seule orientation du réservoir 10. Ceci est notamment avantageux lorsque la forme extérieure du réservoir 10 est sensiblement symétrique mais que l'orifice latéral de sortie 11 n'est pas disposée au centre axial du réservoir 10, comme dans les exemples des figures. En effet, dans ce cas, il est souhaitable d'orienter correctement le réservoir 10 dans l'inhalateur pour un fonctionnement optimal, et la forme asymétrique de l'élément de fermeture permet de remplir cette fonction.

Bien entendu, si le réservoir 10 est symétrique, avec l'orifice latéral de sortie 11 centré axialement, l'élément de fermeture 12 n'a pas besoin d'être asymétrique. Au contraire, un élément de fermeture 12 formant avec le réservoir 10 une unité symétrique permettrait à l'utilisateur d'insérer cette unité dans l'inhalateur dans les deux orientations, sans risques de dysfonctionnement ou distribution de poudre moins efficace.

Avantageusement, comme visible sur la figure 5, l'embout buccal 120 peut comporter une grille 125 en amont de l'orifice de distribution 121.

Selon l'invention, il est prévu une boite de stockage 200 destinée à recevoir l'inhalateur jusqu'à son utilisation.

Cette boite 200 comporte un corps creux borgne 201 ouvert du côté supérieur et fermé par un couvercle 202.

Le corps creux borgne comporte du côté inférieur une paroi de fond 203 supportant au moins une projection axiale 205 s'étendant axialement vers le haut. Dans l'exemple de la figure 1, il y a une seule projection axiale 205.

L'inhalateur est inséré dans la boite de transport 200 avec l'embout buccal 120 dirigé vers la paroi de fond, comme visible sur la figure 1.

Dans sa position de stockage, avec le couvercle fermé, ladite au moins une projection axiale 205 traverse l'embout buccal 120 et la chambre 111 pour venir en contact du réservoir 10, notamment de la partie supérieure de réservoir 10a. Ceci bloque le réservoir 10 dans la position non chargée, et même en cas de chute de la boite 200, le réservoir 10 ne peut pas se déplacer accidentellement dans sa position chargée.

L'ensemble formé de l'inhalateur et de la boite de stockage selon l'invention est simple et efficace. Il est constitué d'un faible nombre de pièces, il est donc peu coûteux à fabriquer et à assembler, et fiable d'utilisation. Il permet une distribution optimale de la poudre de par le mouvement orbital du réservoir lors de l'actionnement, tout en garantissant l'intégrité de la poudre jusqu'à son utilisation.

Diverses modifications sont également possibles pour un homme du métier sans sortir du cadre de la présente invention tel que défini par les revendications annexées.

## Revendications

1. Ensemble comportant :
- un inhalateur de poudre sèche comportant un corps (110) contenant une chambre (111) et un embout buccal (120) pourvu d'un orifice de distribution (121) à travers lequel l'utilisateur inhale, un réservoir (10) contenant une dose de poudre sèche à inhaler et comportant un orifice latéral de sortie (11), ledit réservoir (10) étant déplaçable entre une position non chargée, dans laquelle il est au moins partiellement disposé hors de ladite chambre (111), et une position chargée, dans laquelle il est entièrement disposé dans ladite chambre (111), le diamètre dudit réservoir (10) étant inférieur au diamètre de ladite chambre (111), un organe d'actionnement (130) pour déplacer ledit réservoir (10) de sa position non chargée vers sa position chargée, et
- une boite de stockage (200) comportant un corps creux borgne (201) comportant une paroi de fond (203) supportant au moins une projection axiale (205) s'étendant axialement vers le haut, ladite au moins une projection axiale (205), en position de stockage avec ledit inhalateur disposé dans ladite boite de stockage (200), traversant ledit embout buccal (120) et ladite chambre (111) pour venir en contact dudit réservoir (10) pour bloquer ledit réservoir (10) dans sa position non chargée.

2. Ensemble selon la revendication 1, dans lequel ledit corps creux borgne (201) est ouvert du côté supérieur et fermé par un couvercle (202).

3. Ensemble selon la revendication 1 ou 2, dans lequel ledit réservoir (10) est formé d'une partie supérieure de réservoir (10a) et d'une partie inférieure de réservoir (10b), qui sont assemblées après remplissage avec la dose de poudre.

4. Ensemble selon la revendication 3, dans lequel ladite partie inférieure de réservoir (10b) comporte ledit orifice latéral de sortie (11).

5. Ensemble selon la revendication 3 ou 4, dans lequel ladite au moins une projection axiale (205) de ladite boite de stockage (200) coopère en position de stockage avec ladite partie supérieure de réservoir (10a).

6. Ensemble selon l'une quelconque des revendications précédentes, dans lequel, en position non chargée dudit réservoir (10), ledit orifice latéral de sortie (11) dudit réservoir (10) est obturé par un élément de fermeture (115) solidaire dudit corps (110) de l'inhalateur.

7. Ensemble selon l'une quelconque des revendications 1 à 5, dans lequel ledit réservoir (10) comporte un élément de fermeture (12) déplaçable et/ou déformable par rapport audit réservoir (10) entre une position de fermeture dudit orifice latéral de sortie (11) et une position d'ouverture dudit orifice latéral de sortie (11), ledit élément de fermeture (12) étant en position de fermeture quand ledit réservoir (10) est en position non chargée, et étant en position d'ouverture quand ledit réservoir (10) est en position chargée.

8. Ensemble selon l'une quelconque des revendications précédentes, dans lequel ladite chambre (111) est disposée entre une entrée d'air d'inhalation et ledit embout buccal (120).

9. Ensemble selon l'une quelconque des revendications précédentes, dans lequel des canaux tangentiels (115a, 115b, 115c) amènent un flux d'air d'inhalation de manière tangentielle dans ladite chambre (111), ce qui génère un déplacement orbital dudit réservoir (10) dans ladite chambre (111).

10. Ensemble selon l'une quelconque des revendications précédentes, dans lequel ledit réservoir (10) contient une dose de poudre supérieure à 50 mg, notamment supérieure à 100 mg.

11. Ensemble selon l'une quelconque des revendications précédentes, dans lequel le volume occupé par ledit réservoir (10) dans ladite chambre (111) en position chargée est supérieur à 50% du volume de ladite chambre (111).

12. Ensemble selon l'une quelconque des revendications précédentes, dans lequel le diamètre radial dudit réservoir (10) est supérieur à sa hauteur axiale.

13. Ensemble selon l'une quelconque des revendications précédentes, dans lequel ledit organe d'actionnement (130) est déplaçable axialement par rapport audit corps (110) pour déplacer ledit réservoir (10) de sa position non chargée vers sa position chargée.

14. Ensemble selon la revendication 13, dans lequel ledit organe d'actionnement (130) comporte au moins une rainure oblique recevant au moins un ergot respectif formé sur ledit corps (110) ou sur un élément solidaire dudit corps (110), de sorte que lors de son déplacement axial, ledit organe d'actionnement (130) réalise une rotation dans ledit corps (110).

## Patentansprüche

1. Set, umfassend:
- einen Trockenpulverinhalator, der einen Körper (110), der eine Kammer (111) und ein Mundstück (120), das mit einer Verteileröffnung (121) versehen ist, durch die der Benutzer inhaliert, enthält, ein Reservoir (10), das eine Dosis von zu inhalierendem Trockenpulver enthält und eine seitliche Austrittsöffnung (11) umfasst, wobei das Reservoir (10) zwischen einer nicht geladenen Position, in der es mindestens teilweise außerhalb der Kammer (111) angeordnet ist, und einer geladenen Position, in der es vollständig in der Kammer (111) angeordnet ist, verschiebbar ist, wobei der Durchmesser des Reservoirs (10) kleiner als der Durchmesser der Kammer (111) ist, ein Betätigungsorgan (130), um das Reservoir (10) von seiner nicht geladenen Position in seine geladene Position zu verschieben, umfasst, und
- eine Aufbewahrungsbox (200), die einen Sacklochhohlkörper (201) umfasst, der eine Bodenwand (203) umfasst, die mindestens einen axialen Vorsprung (205) trägt, der sich axial nach oben erstreckt, wobei der mindestens eine axiale Vorsprung (205) in der Aufbewahrungsposition mit dem Inhalator, der in der Aufbewahrungsbox (200) angeordnet ist, das Mundstück (120) und die Kammer (111) durchquert, um mit dem Reservoir (10) in Kontakt zu kommen, um das Reservoir (10) in seiner nicht geladenen Position zu blockieren.

2. Set nach Anspruch 1, wobei der Sacklochkörper (201) an der oberen Seite offen und mit einem Deckel (202) geschlossen ist.

3. Set nach Anspruch 1 oder 2, wobei das Reservoir (10) aus einem oberen Reservoirteil (10a) und einem unteren Reservoirteil (10b) gebildet ist, die nach dem Befüllen mit der Pulverdosis zusammengefügt werden.

4. Set nach Anspruch 3, wobei der untere Reservoirteil (10b) die seitliche Austrittsöffnung (11) umfasst.

5. Set nach Anspruch 3 oder 4, wobei der mindestens eine axiale Vorsprung (205) der Aufbewahrungsbox (200) in der Aufbewahrungsposition mit dem oberen Reservoirteil (10a) zusammenwirkt.

6. Set nach einem der vorhergehenden Ansprüche, wobei in der nicht geladenen Position des Reservoirs (10) die seitliche Austrittsöffnung (11) des Reservoirs (10) von einem Schließelement (115), das mit dem Körper (110) des Inhalators fest verbunden ist, verschlossen ist.

7. Set nach einem der Ansprüche 1 bis 5, wobei das Reservoir (10) ein Schließelement (12) umfasst, das im Verhältnis zu dem Reservoir (10) zwischen einer Schließposition der seitlichen Austrittsöffnung (11) und einer Öffnungsposition der seitlichen Austrittsöffnung (11) verschiebbar und/oder verformbar ist, wobei sich das Schließelement (12) in der Schließposition befindet, wenn sich das Reservoir (10) in der nicht geladenen Position befindet, und sich in der Öffnungsposition befindet, wenn sich das Reservoir (10) in der geladenen Position befindet.

8. Set nach einem der vorhergehenden Ansprüche, wobei die Kammer (111) zwischen einem Inhalationslufteingang und dem Mundstück (120) angeordnet ist.

9. Set nach einem der vorhergehenden Ansprüche, wobei tangentiale Kanäle (115a, 115b, 115c) einen Inhalationsluftstrom tangential in die Kammer (111) bringen, wodurch eine orbitale Verschiebung des Reservoirs (10) in der Kammer (111) entsteht.

10. Set nach einem der vorhergehenden Ansprüche, wobei das Reservoir (10) eine Pulverdosis von mehr als 50 mg, insbesondere von mehr als 100 mg, enthält.

11. Set nach einem der vorhergehenden Ansprüche, wobei das Volumen, das von dem Reservoir (10) in der Kammer (111) in der geladenen Position eingenommen wird, um 50 % größer als das Volumen der Kammer (111) ist.

12. Set nach einem der vorhergehenden Ansprüche, wobei der radiale Durchmesser des Reservoirs (10) größer als seine axiale Höhe ist.

13. Set nach einem der vorhergehenden Ansprüche, wobei das Betätigungsorgan (130) im Verhältnis zum Körper (110) axial verschiebbar ist, um das Reservoir (10) von seiner nicht geladenen Position in seine geladene Position zu verschieben.

14. Set nach Anspruch 13, wobei das Betätigungsorgan (130) mindestens eine schräge Rille umfasst, die mindestens einen jeweiligen Ansatz aufnimmt, der an dem Körper (110) oder an einem mit dem Körper (110) fest verbundenen Element gebildet ist, so dass das Betätigungsorgan (130) bei seiner axialen Verschiebung eine Drehung in dem Körper (110) ausführt.

## Claims

1. A set comprising:
- a dry powder inhaler comprising a body (110) containing a chamber (111) and a mouthpiece (120) provided with a dispensing orifice (121) through which the user inhales, a reservoir (10) containing a dose of dry powder to be inhaled and comprising a lateral outlet orifice (11), said reservoir (10) being displaceable between an unloaded position, in which it is at least partially disposed outside said chamber (111), and a loaded position, in which it is entirely disposed in said chamber (111), the diameter of said reservoir (10) being less than the diameter of said chamber (111), an actuating member (130) for displacing said reservoir (10) from its unloaded position towards its loaded position, and
- a storage box (200) comprising a blind hollow body (201) comprising a bottom wall (203) supporting at least one axial projection (205) which extends axially upwards, said at least one axial projection (205), in the storage position with said inhaler disposed in said storage box (200), passing through said mouthpiece (120) and said chamber (111) so as to come into contact with said reservoir (10) in order to lock said reservoir (10) in its unloaded position.

2. The set as claimed in claim 1, in which said blind hollow body (201) is open on the upper side and closed by a cover (202).

3. The set as claimed in claim 1 or claim 2, in which said reservoir (10) is formed by an upper reservoir portion (10a) and a lower reservoir portion (10b), which are assembled after filling with the dose of powder.

4. The set as claimed in claim 3, in which said lower reservoir portion (10b) comprises said lateral outlet orifice (11).

5. The set as claimed in claim 3 or claim 4, in which said at least one axial projection (205) of said storage box (200) cooperates with said upper reservoir portion (10a) in the storage position.

6. The set as claimed in any one of the preceding claims, in which, in the unloaded position of said reservoir (10), said lateral outlet orifice (11) of said reservoir (10) is closed by a closure element (115) which is secured to said body (110) of the inhaler.

7. The set as claimed in any one of claims 1 to 5, in which said reservoir (10) comprises a closure element (12) which is displaceable and/or deformable with respect to said reservoir (10) between a closed position of said lateral outlet orifice (11) and an open position of said lateral outlet orifice (11), said closure element (12) being in the closed position when said reservoir (10) is in the unloaded position, and being in the open position when said reservoir (10) is in the loaded position.

8. The set as claimed in any one of the preceding claims, in which said chamber (111) is disposed between an inhalation air inlet and said mouthpiece (120).

9. The set as claimed in any one of the preceding claims, in which tangential channels (115a, 115b, 115c) bring a flow of inhalation air into said chamber (111) in a tangential manner, which generates an orbital displacement of said reservoir (10) in said chamber (111).

10. The set as claimed in any one of the preceding claims, in which said reservoir (10) contains a dose of powder greater than 50 mg, in particular greater than 100 mg.

11. The set as claimed in any one of the preceding claims, in which the volume occupied by said reservoir (10) in said chamber (111) in the loaded position is greater than 50% of the volume of said chamber (111).

12. The set as claimed in any one of the preceding claims, in which the radial diameter of said reservoir (10) is greater than its axial height.

13. The set as claimed in any one of the preceding claims, in which said actuating member (130) is axially displaceable with respect to said body (110) in order to displace said reservoir (10) from its unloaded position towards its loaded position.

14. The set as claimed in claim 13, in which said actuation member (130) comprises at least one oblique groove receiving at least one respective lug formed on said body (110) or on an element which is secured to said body (110), in a manner such that during its axial displacement, said actuation member (130) implements a rotation in said body (110).
